# FASCICULE DE BREVET EUROPEEN

(11) **EP 1 869 475 B1**
(45) Date de publication et mention de la délivrance du brevet: **20.08.2008**
(21) Numéro de dépôt: 06721557.4
(22) Date de dépôt: 13.04.2006
(51) Int. Cl.: G01N 33/94, G01N 33/558

(54) **PROCEDE IN-VITRO POUR LA DETECTION ET L'IDENTIFICATION SIMULTANEES D'ANTIBIOTIQUES DE CLASSES DIFFERENTES ET TROUSSE DE DIAGNOSTIC CORRESPONDANTE**
IN-VITRO-VERFAHREN ZUM GLEICHZEITIGEN NACHWEISEN UND IDENTIFIZIEREN VON ANTIBIOTIKA UNTERSCHIEDLICHER KLASSEN SOWIE ENTSPRECHENDER DIAGNOSTISCHER KIT
IN VITRO METHOD FOR SIMULTANEOUSLY DETECTING AND IDENTIFYING ANTIBIOTICS OF DIFFERENT CLASSES AND CORRESPONDING DIAGNOSTIC KIT

(30) Priorité: 14.04.2005 EP 05447079
(43) Date de publication de la demande: 26.12.2007
(73) Titulaire: Unisensor S.A., 4020 Wandre (BE)
(72) Inventeur: GRANIER, Benoît, 4120 Rotheux-Rimiere (BE)
(74) Mandataire: pronovem
(86) Numéro de dépôt international: PCT/BE2006/000036
(87) Numéro de publication internationale: WO 2006/108248

(56) Documents cités:
- WO-A-99/67416
- WO-A-03/048770
- VERHEIJEN R ET AL: "DEVELOPMENT OF A ONE STEP STRIP TEST FOR THE DETECTION OF SULFADIMIDINE RESIDUES" ANALYST, ROYAL SOCIETY OF CHEMISTRY, LONDON, GB, vol. 123, no. 12, décembre 1998 (1998-12), pages 2437-2441, XP009059768 ISSN: 0003-2654
- ROWE C A ET AL: "AN ARRAY IMMUNOSENSOR FOR SIMULTANEOUS DETECTION OF CLINICAL ANALYTES" ANALYTICAL CHEMISTRY, AMERICAN CHEMICAL SOCIETY. COLUMBUS, US, vol. 71, no. 2, 15 janvier 1999 (1999-01-15), pages 433-439, XP000825701 ISSN: 0003-2700
- GOLEMI-KOTRA DASANTILA ET AL: "Resistance to beta-lactam antibiotics and its mediation by the sensor domain of the transmembrane BlaR signaling pathway in Staphylococcus aureus." JOURNAL OF BIOLOGICAL CHEMISTRY, vol. 278, no. 20, 16 mai 2003 (2003-05-16), pages 18419-18425, XP002397480 ISSN: 0021-9258

## Description

### Objet de l'invention

La présente invention se rapporte à un procédé consistant à faire réagir simultanément en un seul mélange réactionnel, un ensemble de diverses molécules biologiques de reconnaissance, capables de détecter spécifiquement plusieurs analytes distincts avec une sensibilité élevée et de déterminer la classe d'analytes à laquelle appartient chacun des composés détectés, sans que le principe de reconnaissance spécifique d'une classe d'analytes donnée ne puisse interférer sur le principe de reconnaissance spécifique d'une autre classe d'analytes.

L'invention concerne également la trousse de diagnostic spécialement conçue pour la mise en oeuvre du procédé.

### Arrière-plan technologique

Un principe fondamental qui gouverne les bonnes pratiques de surveillance et de contrôle de la chaîne alimentaire impose de réaliser des analyses de contrôle le plus en amont possible de la production pour permettre d'identifier et d'isoler le plus rapidement possible les denrées suspectées d'être contaminées.

En règle générale, lorsque l'on procède à des tests de dépistage souvent appelé "tests de screening", un échantillon détecté positif au cours d'une première analyse de contrôle est seulement supposé être effectivement positif et il doit subir obligatoirement un second test dit "de confirmation". A contrario, si un test initial de dépistage donne une réponse négative, elle est suffisante et aucune analyse ultérieure ne doit encore confirmer le résultat⁽¹⁾.

Une première conséquence de cette règle est que la première méthode de dépistage doit pouvoir couvrir la détection d'un maximum de composés. Les tests de screening ou de dépistage doivent donc préférentiellement et logiquement être des "tests multi-analytes".

Une seconde conséquence de cette règle est qu'il est important de connaître la classe à laquelle appartient le composé retrouvé dans un échantillon positif lors du test de screening de manière à pouvoir orienter directement vers la méthode de confirmation qui est en générale très particulière car il est recommandé d'isoler et d'identifier le composé concerné.

Une troisième conséquence de cette règle est qu'un test de screening ne peut pas donner de résultats de type "faux négatif" car ceux-ci échapperont dès lors à l'analyse à ce stade préliminaire et ne seront pas confirmés ultérieurement.

Actuellement, on connaît diverses méthodes pour la détection des antibiotiques :
- les tests microbiologiques qui mesurent le pouvoir inhibiteur d'un échantillon sur la croissance d'une souche bactérienne. Ce type de test demande un temps d'incubation relativement long (entre 3 et 16 heures) avant l'obtention du résultat. En général, ces tests (Delvotest^{®} SP, BRT Test, Copan^{™}, Eclipse^{™}, Valio^{™}) ont la capacité de reconnaître plusieurs classes d'antibiotiques simultanément car les souches bactériennes utilisées sont souvent sensibles à plusieurs composés de classes différentes. Cependant, ce type de test ne permet pas d'identifier à quelle classe précise appartient le composé d'antibiotique testé ;
- les tests *in vitro* fonctionnent, dans le cas de la détection de petites molécules, selon un principe de compétition entre le composé à rechercher qui est présent dans l'échantillon et un compétiteur marqué qui a été volontairement introduit dans l'échantillon pour un seul et même site de reconnaissance qui peut être constitué soit par un récepteur, soit par un anticorps. Dans une formulation de type ELISA *(Enzyme Linked Immuno Sorbent Assay)* ou RIA *(Radio Immuno Assay)* / RRA *(Radio Receptor Assay),* le temps requis pour une analyse est de l'ordre de 2 à 6 heures. Certaines de ces méthodes, en particulier les méthodes RRA de laboratoire, permettent la détection simultanée de plusieurs composés de classes différentes. Cependant dans ce cas, la méthode ne permet pas d'identifier à quelle classe appartient le composé ayant donné une réponse positive ;
- les méthodes physico-chimiques, plus complexes, qui permettent d'isoler et d'identifier le composé recherché, sont, quant à elles, depuis peu, principalement des méthodes où un système de séparation chromatographique est couplé à un système de détection en spectrométrie de masse (GC/MS ou LC/MS). Ces méthodes demandent l'adaptation de protocoles particuliers pour chaque composé distinct à identifier. Tantôt, un seul composé ou une partie des composés d'une même classe peuvent être analysés simultanément, tantôt c'est l'ensemble des composés d'une même classe qui peuvent être détectés mais cela n'est jamais possible avec des composés de classes distinctes. En effet, le principe de séparation chromatographique est caractéristique des propriétés physico-chimiques d'un composé donné, ces dernières étant souvent différentes d'une classe à l'autre. Dans le cas où l'opérateur ne connaît pas le type de composé à identifier, il doit passer en revue autant de méthodes qu'il y a de classes de composés.

Depuis quelques années, on assiste également au développement de méthodes beaucoup plus rapides. Ces méthodes, dites "RAPID TESTS" sont utilisées pour effectuer un dépistage rapide sur un grand nombre d'échantillons. En général ces méthodes exploitent la reconnaissance des composés recherchés vis-à-vis d'une molécule biologique selon un principe de compétition. Pour rendre l'analyse rapide et pratique, ces types de tests fonctionnent à l'aide de dispositifs membranaires à flux latéral (Tetrasensor^{™}, SNAP^{®}, Beta-STAR^{™}, ROSA). Ces tests se déclinent selon diverses classes d'antibiotiques mais aucun produit connu à ce jour ne permet de détecter en une seule opération des composés appartenant à des classes différentes.

S'il est raisonnable d'envisager et d'imposer au secteur agroalimentaire des analyses de dépistage primaire, le secteur concerné sera demandeur d'une analyse la plus complète possible et qui puisse de préférence identifier un maximum de composés suspects. Il est en effet plus pratique et économique de réaliser un seul test multiple à partir d'un seul échantillon plutôt que de devoir réaliser un test particulier pour chaque composé particulier. Cette pratique est lourde en temps, en gestion des échantillons et en coût. Elle constitue un frein à la bonne gestion et au contrôle efficace des denrées alimentaires.

Le contrôle rencontre donc à ce stade une limite importante à l'efficacité qui est caractérisée par l'absence de tests multi-analytes qui permettraient de détecter en une seule opération et en moins de 10 minutes par exemple, l'ensemble des composés de plusieurs classes d'analytes.

En particulier, le secteur agroalimentaire serait intéressé par un nouveau procédé permettant d'envisager en une seule opération l'analyse de composés appartenant à au moins deux classes différentes d'antibiotiques.

Le type d'antibiotique qui peut être administré à des animaux peut varier selon qu'il s'agit d'une application thérapeutique ou prophylactique, selon l'espèce animale, le germe à combattre, les pratiques vétérinaires, la législation en vigueur, les moyens disponibles ou encore les régions géographiques. Dans le cas de certains traitements particuliers, on utilise un mélange de médicaments. En règle générale, le praticien utilise des produits antibiotiques choisis parmi tous les composés commercialement disponibles selon son appréciation de la meilleure efficacité.

Les principales classes d'antibactériens et d'antibiotiques utilisés sont : les pénicillines et céphalosporines, les tétracyclines, les sulfamides, les aminoglycosides et aminocyclitols, les macrolides, les chloramphénicols ou autres peptides, ionophores, nitrofurannes, quinolones, carbadox, etc. Toutes ces classes regroupent un ensemble très vaste de composés chimiquement différents.

On pense que l'utilisation, parfois intensive, d'antibiotiques en médecine vétérinaire et en production agricole pourrait être à l'origine de l'émergence de souches bactériennes devenues résistantes aux antibiotiques. Pour préserver la santé humaine et légiférer en la matière, de nombreux pays (Union européenne, USA, Canada, etc.) ont établi des limites maximales autorisées (MRL) pour les résidus d'antibiotiques dans les denrées alimentaires⁽²⁾. Ces MRLs fixent en quelque sorte la limite entre un échantillon positif et un échantillon négatif, c'est-à-dire entre un échantillon refusé et un échantillon accepté.

Par ailleurs, la Décision de la Commission du 12 août 2002 a fixé les limites de performances minimales requises (LPMR) applicables aux méthodes d'analyse à utiliser pour l'examen des échantillons et a défini des critères communs pour l'interprétation des résultats⁽³⁾.

Il est entendu que seules les techniques d'analyse dont on peut démontrer, sur base de preuves identifiables, qu'elles sont validées et ont un taux de faux conformes inférieurs à 5% au niveau considéré doivent être appliquées aux fins de dépistage conformément à la Directive 96/23/CE.

En 1995, en moyenne un pour cent de tous les échantillons analysés par rapport à leur contenu en antibiotiques présentaient une teneur supérieure à la MRL et donnaient une réponse positive. Lorsque ces résultats positifs ont été confirmés, les produits le plus souvent rencontrés étaient des pénicillines et des tétracyclines ⁽⁴⁾.

### Etat de la technique

Un système de reconnaissance des molécules de tétracycline est décrit dans la demande de brevet de la Demanderesse WO-A-03/048770 et intitulée "Procédé pour effectuer un diagnostic *in vitro* au moyen de mécanismes de régulation génétique et trousse de diagnostic correspondante". Cette méthode ne permet pas de détecter autre chose que des tétracyclines. Dans une formulation préférée, les réactifs sont constitués d'un récepteur tetR, isolé du mécanisme de régulation génétique d'E. coli pour la résistance aux tétracyclines, d'un anticorps capable de reconnaître le récepteur et d'une préparation de protéine-A conjuguée à des particules d'or colloïdales. D'autre part, le système récupérateur est un fragment d'ADN spécifique et biotinylé qui est immobilisable sur une molécule d'avidine fixée sur une membrane de nitrocellulose. C'est pendant l'incubation en présence de l'échantillon que le récepteur et le fragment d'ADN interagissent uniquement en l'absence de tétracycline. Dans ce cas, le fragment d'ADN forme un complexe avec le récepteur et un signal généré par les particules d'or fixées au récepteur apparaît. Dans le cas contraire le récepteur qui a reconnu préalablement une molécule de tétracycline n'est plus capable de se lier au fragment d'ADN et en conséquence aucun signal n'apparaît.

Un système de reconnaissance des ß-lactames est décrit dans le brevet WO-A-99/67416 intitulé "Procédé pour la détermination d'antibiotiques à noyaux ß-lactame dans un liquide biologique". Selon ce procédé, il n'est pas possible de détecter autre chose que des β-lactames. Dans une formulation préférée, les inventeurs décrivent d'une part un récepteur isolé de Bacillus licheniformis*,* purifié et couplé chimiquement à des molécules de biotine, auquel est associé une préparation d'anticorps anti-biotine conjugués à des particules d'or colloïdales, et d'autre part un conjugué formé d'une céphalosporine couplée à une immunoglobuline humaine et immobilisé sur une membrane de nitrocellulose. Dans le cas où l'échantillon ne contient pas de β-lactame, le récepteur se fixe à l'antibiotique immobilisé et un signal généré par les particules d'or fixées au récepteur-biotine, par l'intermédiaire d'un anticorps anti-biotine couplé à l'or, apparaît. Dans le cas contraire, si le récepteur est inhibé au cours d'une incubation préalable avec l'échantillon à tester, il n'est plus capable de se lier à l'antibiotique immobilisé et en conséquence aucun signal n'apparaît.

On connaît aussi un système de reconnaissance rapide des sulfamides décrit par R. Verheijen *et al.*⁽⁵⁾ et intitulé "Development of a one step strip test for the détection of sulfadimidine residues". Ce système utilise un principe similaire à celui de la détection des β-lactames mentionné ci-dessus. D'une part, un anticorps spécifique des sulfadimidines est conjugué à des particules d'or colloïdales et c'est une sulfadimidine qui est conjuguée à de l'ovalbumine et immobilisée sur une membrane de nitrocellulose. Si l'échantillon contient des sulfadimidines reconnues par l'anticorps, celui-ci forme un complexe qui est incapable de rencontrer ultérieurement le composé immobilisé et en conséquence ne pourra pas s'y fixer. Dans le cas contraire en absence de sulfadimidine dans l'échantillon, l'anticorps conjugué pourra reconnaître le sulfadimidine immobilisée et un signal coloré apparaîtra. Cet article n'indique pas la spécificité de ce test.

Toutes les méthodes et procédés rapides en flux latéral, connus à ce jour, s'appliquent uniquement à la détection d'une seule et même classe de composés et il n'existe pas à ce jour de procédé permettant de détecter en une seule opération tous les composés appartenant à aux moins deux classes distinctes d'antibiotiques. Les raisons principales en sont une incompatibilité technique de réunir de manière indépendante et non-interférente, dans un seul et même procédé, tous les réactifs nécessaires à la détection de chacune des classes. La seconde difficulté réside ensuite dans la manière d'arriver à l'identification de la classe à laquelle appartient le composé détecté.

En résumé, les systèmes rapides connus pour la détection de petites molécules (MW < 2000) fonctionnent sur le principe de compétition qui demande l'utilisation de deux éléments particuliers que sont, d'une part, une molécule qui puisse reconnaître spécifiquement le composé à détecter, cette molécule étant en général un récepteur bactérien ou une immunoglobuline (anticorps), et d'autre part, un compétiteur pour le site de reconnaissance. Selon la méthode choisie, un des éléments est immobilisé sur un support et l'autre élément est marqué. Dans certains cas (format 1, voir ci-après), c'est la molécule de reconnaissance qui est marquée et un analogue de l'analyte qui est immobilisé ; dans d'autres cas (format 2, voir ci-après), c'est un analogue de l'analyte qui est marqué et c'est la molécule de reconnaissance qui est solidaire d'une fraction insoluble.

L'originalité du système de détection des tétracyclines se caractérise par le fait que le récepteur comprend deux sites de reconnaissance interdépendants et en conséquence, le compétiteur n'est pas un analogue de l'analyte recherché mais un fragment d'ADN capable de se fixer au second site de reconnaissance du même récepteur.

Dans toutes les méthodes dites "RAPID TESTS", l'appréciation est faite en comparant l'intensité du signal obtenu à la zone "test", c'est-à-dire à l'endroit ou l'élément récupérateur du récepteur actif est immobilisé par rapport à l'intensité d'un autre signal qui est obtenu à la zone "contrôle", c'est-à-dire à l'endroit où d'autres réactifs (ou l'excédent des réactifs) sont récupérés. En général, lorsque l'intensité à la zone "test" est plus marquée qu'à la zone "contrôle", le résultat est négatif pour l'élément recherché.

Dans tous les cas relatifs au dosage des molécules de ß-lactames, la molécule de reconnaissance est obtenue à partir de préparation de Bacillus et dans tous les cas, cette molécule est marquée après purification. Pour réaliser ce marquage, il y a lieu de procéder à une modification chimique de surface. Par exemple, elle peut être couplée chimiquement à une enzyme, comme c'est le cas avec le récepteur de *Bacillus stéarothermophillus* couplé à de la peroxydase (EP-A-0 593112 et US-A-5,434,053 de Giest Brocades) ou à une biotine, comme c'est le cas avec le récepteur de *Bacillus licheniformis* couplé à une biotine (WO-A-99/67416 et US-A-6,524,804 de U.C.B. S.A.) ou encore avec le récepteur de *Bacillus stéarothermophillus* directement conjugué à de l'or colloïdal (US-A-6,475,805 de Charm Inc.). A l'exception de la méthode Tetrasensor^{™}, dans les autres cas connus à ce jour, il est nécessaire de modifier chimiquement la molécule de reconnaissance pour en obtenir un complexe coloré. En conséquence, il faut la purifier et en modifier certains des substituants de surface, au risque d'en modifier une propriété importante de fonctionnalité ou de stabilité.

Dans les autres cas connus à ce jour, il est en conséquence généralement impossible de travailler soit avec des extraits bruts non purifiés soit avec des récepteurs qui ne seraient pas modifiés chimiquement.

Par ailleurs, le type de marquage qui est exploité dans le dosage "tétracycline" n'est pas compatible avec le dosage des β-lactames car, dans celui-ci, c'est une immunoglobuline qui est immobilisée à la zone "contrôle" dans tous les cas renseignés. Dans le cas du document WO-99/67416, c'est aussi une immunoglobuline qui sert de protéine d'ancrage pour immobiliser le compétiteur à la zone "test". L'utilisation de la protéine-A, comme dans le dosage des tétracyclines, provoquerait inévitablement un marquage non spécifique sur une ou sur les deux lignes de capture nécessaires à la détection des β-lactames.

Dans un autre ordre d'idée, lorsque l'on doit recourir à l'utilisation de l'ensemble avidine-biotine pour faciliter soit la fixation sur le support, soit le marquage, l'ensemble avidine-biotine ne peut être utilisé dans une méthode donnée que pour une seule reconnaissance spécifique. En effet si une avidine constitue un point d'ancrage, toutes les molécules biotinylées pourront venir s'y fixer. Dans un tel cas, il est notamment irréalisable d'envisager un test pour la détection commune des ß-lactames et des tétracyclines qui serait basé par exemple sur la réunion des deux principes de dosage décrits dans le document WO-A-99/67416 et dans le document WO-A-03/048770. En effet, dans le premier cas le récepteur est biotinylé pour ensuite être marqué à l'aide d'antibiotine-or et dans le second cas de l'avidine est immobilisée pour récupérer un fragment d'ADN biotinylé. La réunion de ces deux principes conduirait encore à un autre conflit entre les deux systèmes indépendants utilisant les couples avidine-biotine. Dans une réunion des deux systèmes existants, le récepteur biotinylé du système ß-lactame se fixerait dans l'avidine nécessaire à récupérer l'ADN du système tétracycline et par voie de conséquence occasionnerait un marquage non spécifique indépendamment de la teneur en tétracycline dans l'échantillon. De plus la présence d'antibiotine-or dans la phase mobile, interférerait avec l'ADN biotinylé empêchant celui-ci de rencontrer l'avidine nécessaire à sa capture ce qui aboutirait encore à un autre conflit.

Dans un troisième ordre d'idée, la réunion de deux systèmes indépendants de reconnaissance demanderait un système de lecture et d'interprétation du résultat assez complexe où chacun des marqueurs doit s'apprécier par l'intensité relative à une référence interne. Il y aurait en conséquence deux bandes "test" ("beta" et "tetra") et deux bandes "contrôle". Cette vision ne rend l'analyse ni simple, ni pratique. Dans le cadre d'une réunion des deux tests, il vaudrait mieux apprécier chacune des intensités des tests par comparaison entre elles. Dans une telle pratique, la zone "test" pour l'analyte n°1 deviendrait la zone "contrôle" pour l'analyte n°2 et vice versa. Dans le cas ou les deux classes d'analytes concernées seraient présentes en même temps dans un même échantillon, cette approche générerait pas ou peu de signal. C'est rarement le cas mais s'il se présentait, pour éviter une interprétation difficile dans le cas d'une double contamination par des ß-lactames et tétracyclines, il convient de préconiser l'ajout d'une référence unique sous la forme d'une seule ligne contrôle permettant plus aisément d'apprécier l'intensité relative des deux signaux tests. Il est évident que cette ligne unique de contrôle deviendrait nécessaire dans le cas où plus de deux tests seraient intégrés dans un même procédé.

Dans un quatrième ordre d'idée, les matériaux choisis expressément et préférentiellement pour constituer le dispositif de tigette de la trousse de diagnostic doivent être compatibles pour l'analyse simultanée des deux classes d'analytes. En effet dans une modalité d'exécution préférée décrite dans WO99/67416, il est recommandé d'utiliser la membrane de type "Leukosorb^{®}" qui n'est pas compatible avec l'approche que nous préconisons pour le test des tétracyclines et ne conviendrait pas pour un dosage multi-analyte. En effet, très étrangement, dans une formulation ou les deux récepteurs sont marqués à l'aide de leur anticorps respectif, il est impossible d'obtenir un point de coupure net par ajout de β-lactames lorsque les réactifs rencontrent la membrane Leukosorb^{®} avant d'atteindre le point de capture. Cette membrane, dont l'efficacité a été démontrée pour la purification du lait et l'utilisation en flux latéral, est la cause de signaux de reconnaissance non spécifiques lorsque le marquage est réalisé à l'aide d'anticorps et de protéine-A conjuguée à de l'or colloïdal.

Dans un cinquième ordre d'idée, le choix des récepteurs impliqués dans la reconnaissance de l'ensemble des composés de chacune des classes est important. Ce choix n'est pas uniquement déterminé par les performances de reconnaissance des composés mais également par des critères de stabilité, de structure, de réactivité antigénique, de composition en acides aminés, etc.

En conclusion, sur base des procédés connus, il est a *priori* impossible de rassembler dans un seul et même procédé au moins deux procédés indépendants, par exemple pour la reconnaissance par des récepteurs aux β-lactames et aux tétracyclines, sans envisager des modifications substantielles autorisant à réunir tous les éléments nécessaires en s'assurant que le système de reconnaissance d'un composé n'interfère pas avec le système de reconnaissance d'un autre composé.

### Buts de l'invention

La présente invention vise à fournir un nouveau procédé de diagnostic permettant de détecter simultanément un ensemble -en principe illimité- de composés pouvant appartenir à au moins deux classes distinctes d'analytes et de caractériser la classe à laquelle appartient effectivement un composé détecté.

En particulier, l'invention vise à démontrer la compatibilité technique et pratique de réunir en un seul et même procédé au moins deux mécanismes de détection sans que le fonctionnement de l'un d'entre eux ne puisse interférer avec le fonctionnement de l'autre mécanisme.

Un but complémentaire de l'invention est de démontrer la faisabilité technique d'un dosage multi-analytes qui peut s'opérer rapidement, par exemple en moins de 30 minutes et en une seule et même étape d'analyse à l'aide d'un seul et même échantillon.

Un but complémentaire de l'invention est de fournir un procédé et une trousse de diagnostic *in vitro* pour la détection et le dosage simultanés d'au moins deux classes d'analytes.

### Principaux éléments caractéristiques de l'invention

Un premier objet de la présente invention, énoncé dans les revendications respectives 1, 3, 4 et 10, se rapporte à une trousse de diagnostic pour la détection ou la quantification simultanée et spécifique d'au moins deux analytes appartenant à des classes d'antibiotiques différentes, à savoir au moins deux classes parmi les β-lactames, les tétracyclines et les sulfamides, caractérisée en ce qu'elle comprend, de manière générique, par exemple :
- d'une part, un seul mélange réactionnel, de préférence sous forme d'une solution ou d'un lyophilisat, comprenant au moins deux molécules biologiques différentes, capables chacune de reconnaître, respectivement, simultanément et spécifiquement, un analyte déterminé présent dans un échantillon pouvant contenir des analytes appartenant auxdites classes distinctes d'analytes ;
- et d'autre part, un système récupérateur sous la forme d'un support solide unique auquel se trouvent fixés, en des emplacements distincts et connus dans l'espace, des ligands respectifs capables de récupérer spécifiquement, sélectivement et exclusivement chacune desdites molécules biologiques contenues dans ledit mélange réactionnel, de manière à identifier par l'emplacement de la récupération sur ledit support, le type de classe à laquelle appartient chacun des analytes présents dans l'échantillon.

Selon le cas, soit l'élément récupérateur immobilisé est un analogue compétiteur de la substance recherchée et le récepteur en solution est marqué, soit l'analogue compétiteur en solution est marqué et l'élément récupérateur immobilisé est le récepteur. Des systèmes mixtes peuvent aussi coexister selon l'invention.

Des modalités préférées de l'invention sont décrites dans les revendications secondaires 2, 5 à 9 et 11-12.

Un second objet de l'invention, indiqué dans la revendication 13, concerne un procédé de mise en oeuvre d'une trousse de diagnostic selon l'une quelconque des revendications 1 à 12, caractérisé par les étapes suivantes :
- on met en contact le mélange réactionnel précité avec un échantillon à caractériser pour obtenir une solution qu'on laisse incuber à une température comprise entre 30°C et 50°C pendant 3 à 15 minutes ;
- on plonge une tigette portant le système récupérateur précité dans la solution obtenue et on laisse incuber pendant 3 à 15 minutes ;
- on interprète le résultat sur la tigette soit visuellement à l'oeil, soit au moyen d'un lecteur optique de tigette.

Dans les formes d'exécution préférées de la présente invention, on préconise d'éviter d'une part la purification et d'autre part la modification chimique du récepteur en ayant recours par exemple à des anticorps anti-récepteur qui seraient marqués soit directement à l'or colloïdal soit préférentiellement à l'aide de protéine-A couplée à l'or colloïdal. D'autre part, dans une idée de multiplicité de détection, la protéine-A marquée à l'or colloïdal servirait de marqueur générique pour tous les anticorps introduits dans la solution.

Afin de préserver au maximum les fonctionnalités des récepteurs et anticorps utilisés dans la présente invention, un principe adopté est qu'aucun marquage par modification chimique n'a lieu. En conséquence la présente invention exploite les récepteurs bactériens dans leur état le plus naturel possible. Ces récepteurs sont marqués à l'aide d'anticorps qui seront eux- mêmes reconnus par une protéine-A conjuguée à de l'or colloïdal. Le cas échéant, c'est donc exclusivement la protéine de marquage qui sera couplée à l'or mais pas les molécules sensibles pour la reconnaissance des analytes concernés.

Dans cette approche, tous les groupements fonctionnels des molécules sensibles pour la reconnaissance sont donc préservés. Cet avantage prend toute son importance lorsqu'il s'agit d'exploiter l'activité de récepteurs dont le mécanisme de reconnaissance dépend de certaines chaînes latérales comme les résidus lysines par exemple. C'est d'autant plus important que lorsqu'une modification chimique à lieu, dans la plupart des cas renseignés, la modification implique les résidus NH₃⁺ des chaînes latérales des lysines. En conclusion, un couplage chimique sur les lysines peut altérer des lysines du site actif et rendre ainsi la préparation partiellement inactive.

Le marquage par l'intermédiaire d'anticorps présente la souplesse d'un marquage non figé. En effet, un marquage chimique implique une liaison covalente et irréversible alors qu'une fixation d'anticorps est réversible et sujette à un équilibre qui peut être déplacé selon les forces en action. Un autre avantage est de pouvoir moduler l'étape de marquage dans un deuxième temps. En effet, dans une deuxième formulation de la présente invention, c'est après la phase de reconnaissance de l'analyte par le récepteur, que ce soit l'analyte libre de l'échantillon ou l'analyte immobilisé pour la récupération, que l'anticorps reconnaît le récepteur.

Pour diverses raisons économiques ou de stabilité, mais aussi pour une parfaite intégration dans un test multi-analytes, la présente invention préconise préférentiellement l'utilisation d'un récepteur des ß-lactames isolé à partir de *Staphylococcus aureus*⁽⁶⁾*.*

En effet, étant originaire de la souche bactérienne la plus performante à l'égard de la lutte contre les antibiotiques et les β-lactames en particulier, ce récepteur utilisé *in vitro* a démontré des capacités exceptionnelles de reconnaissance de l'ensemble de composés des β-lactames, tant pour les pénicillines que pour les céphalosporines (voir réf.⁽⁶⁾ et résultats repris en exemple).

Un autre avantage de ce récepteur est d'avoir un point isoélectrique (pI) basique. Ceci offre la possibilité de pouvoir le purifier très facilement à partir d'un extrait brut de cellules pour en obtenir des anticorps.

Par ailleurs, notre choix a également été déterminé par l'observation d'une très bonne réponse immunitaire chez le lapin, très spécifique et n'occasionnant aucune réponse non spécifique avec le reste de l'ensemble des réactifs impliqués dans le dosage multi-analytes.

Selon la présente invention, on est parvenu à formuler un procédé dans lequel il est possible de réunir dans une seule et même opération tous les éléments nécessaires à la détection commune de plusieurs composés de classes différentes en utilisant des mécanismes de reconnaissance totalement différents.

La présente invention offre l'avantage supplémentaire de proposer une formulation d'un test multi-analytes fiable et économique. En effet, il n'est pas nécessaire de purifier les récepteurs, ni les préparations d'anticorps et de plus les récepteurs ne sont pas modifiés chimiquement, ce qui devrait préserver l'intégralité de leur réactivité ainsi que leur stabilité.

### Brève description des figures

La figure 1 représente schématiquement un exemple de positionnement des éléments récupérateurs selon l'invention sur un support solide de nitrocellulose dans le cas du dosage simultané de seulement deux antibiotiques, une zone de contrôle fixe étant également prévue.

La figure 2 représente schématiquement un exemple de positionnement des éléments récupérateurs selon l'invention sur un support solide de nitrocellulose dans le cas du dosage simultané de tétracyclines, de β-lactames et de sulfamides, une zone de contrôle fixe étant également prévue.

### Description de modes d'exécution préférés de l'invention

### Remarque préliminaire

Comme évoqué dans l'état de la technique, un test de compétition envisagé selon la présente invention peut notamment se présenter sous deux formats différents. Dans le premier cas (format 1), ce sont les récepteurs qui sont conjugués à des particules d'or colloïdales et les composés compétiteurs, analogues de la substance recherchée, qui servent d'éléments récupérateurs en étant immobilisés au point de capture spécifique. Dans le second cas (format 2), ce sont les composés compétiteurs, analogues de la substance recherchée, qui sont conjugués à des particules d'or colloïdales et ce sont les récepteurs qui servent d'éléments récupérateurs en étant immobilisés au point de capture spécifique. Dans certains cas de détection multi-analytes, on peut aussi avoir un système mixte (format 1 et 2 présents).

### Exemple N°1 : Dosage simultané des tétracyclines et des β-lactames à l'aide de récepteurs spécifiques (format 1 pour les deux détections)

Le procédé utilise un mélange réactionnel contenant les deux récepteurs respectifs des β-lactamines et tétracyclines ainsi que leurs réactifs et un élément récupérateur auquel se trouvent immobilisés, à des endroits précis et distincts, des ligands spécifiques de ces récepteurs.

### Préparation du mélange réactionnel

Pour la détection des β-lactames, le mélange réactionnel contient le récepteur spécifique de reconnaissance des β-lactames, son anticorps spécifique et une préparation de protéine-A conjuguée à de l'or colloïdal. Pour la détection des tétracyclines, le mélange réactionnel contient le récepteur des tétracyclines, son anticorps spécifique, un fragment d'ADN spécifique conjugué à de la biotine et une préparation de protéine-A conjuguée à de l'or colloïdal.

Le récepteur β-lactame est obtenu selon la méthode décrite par Golemi-Kotra et al.⁽⁶⁾. Le récepteur non purifié est filtré sur membrane Millex HV (Millipore, Inc, USA) avant d'être conservé à 4°C en présence de glycérol 50% v/v. Le récepteur tétracycline est obtenu selon la méthode décrite dans la demande de brevet WO-A-03/048770.

Les deux préparations d'anticorps sont obtenues selon la méthode décrite dans Kachab et al.⁽⁷⁾. Ces préparations d'anticorps ont été obtenues par injection d'une préparation de récepteur purifié jusqu'à homogénéité protéique selon Golemi-Kotra et al.⁽⁶⁾ pour le récepteur aux β-lactames et selon la demande WO-A-03/048770 pour le récepteur aux tétracyclines. Les deux préparations d'anticorps sont utilisées préférentiellement non purifiées ce qui signifie que c'est du sérum non purifié qui est directement ajouté au mélange réactionnel. Selon une seconde modalité préférée, les anticorps spécifiques, de type polyclonal ou monoclonal, sont purifiés selon les méthodes connues de l'homme de métier pour ensuite être couplés soit directement soit indirectement à des particules d'or colloïdal selon la méthode de Frens⁽⁸⁾.

Le fragment d'ADN est obtenu chez Eurogentec SA, Belgique, selon la séquence et la préparation reprises dans la demande de brevet WO-A-03/048770. Dans ce cas précis, le fragment d'ADN n'est pas immobilisé à un point de capture sur une membrane de nitrocellulose mais il est directement intégré au mélange réactionnel. Ce fragment sera récupéré *in testo* à l'aide de son extrémité biotinylée sur de l'avidine immobilisée à un point de capture (cf. ci-après).

L'ensemble des réactifs nécessaires sont introduits sans aucune modification chimique ni substitution de manière à préserver l'intégralité des propriétés d'activité et de stabilité. Ces molécules peuvent être purifiées ou non purifiées ce qui présente un avantage économique évident.

Dans l'exemple précis, le mélange réactionnel contient :
- 5 parts de récepteur β-lactame (RSA) à une concentration de 360 nM ;
- 5 parts de récepteur tétracycline (TetR) à une concentration de 4,2 µM ;
- 1 part de sérum anti-RSA diluée 4 x en tampon NaPi 50 mM, NaCl 150 mM, pH 7,8 ;
- 1 part de sérum anti-TetR diluée 4 x en tampon NaPi 50 mM, NaCl 150 mM, pH 7,8 ;
- 1 part d'acide nucléique à une concentration de 25 µM en NaCl 690 mM ;
- 15 parts de protéine-A Or de 40 nm à une DO=10 à 520nm ;
- 22 parts de tampon Hepes 120 mM, pH 8 , BSA 2%, Dextran 1%, sucrose 5%.
Ce mélange est soit préparé extemporanément soit lyophilisé pendant 20 heures.

### Le système récupérateur

Le système récupérateur est constitué d'une membrane de nitrocellulose sur laquelle se trouvent fixés, à des endroits distincts, les éléments capables de former un complexe avec les molécules de reconnaissance du mélange réactionnel. Ces éléments sont :
- pour le signal dit "beta" (pour β-lactame) , un antibiotique, de préférence de type pénicilline ou céphalosporine, immobilisé sur une molécule ne présentant aucune réactivité particulière avec la protéine-A ou la biotine ou encore l'avidine. Dans un mode d'exécution préféré de la présente invention, on utilise une ampicilline immobilisée sur une β-lactoglobuline ;
- pour le signal dit "tetra" (pour tétracycline), une avidine capable de reconnaître un fragment d'ADN présentant à une extrémité une molécule de biotine. De préférence, on utilisera une avidine d'oeuf.

Les zones de capture "beta" et "tetra" peuvent être localisées successivement l'une avant l'autre, ou inversement, sans préférence particulière pour autant qu'elle soit disposées à des emplacements spatialement distincts mais connus. C'est en effet cette position distincte qui permettra de repérer le type d'antibiotique présent dans la solution d'analyse. Si les deux systèmes de capture étaient réunis en un seul endroit, le dosage n'en serait pas altéré mais il ne serait pas possible de déterminer quel type de composé est effectivement présent.

### Préparation de β-lactoglobuline conjuguée à une ampicilline

La β-lactoglobuline est choisie car c'est une protéine du lait qui contient beaucoup de résidus lysine (10%) et la structure tridimensionnelle indique que la plupart des lysines présentent leur extrémité NH₂ vers l'extérieur de la protéine (Pubmed, structure, 1GXA).

100 mg de ß-lactoglobuline et 15 mg de 2-iminothiolane sont incubés en tampon NaPi 100mM, pH 8,5 dans un volume réactionnel de 4 ml pendant 60 minutes à 25°C. Le mélange est ensuite déposé sur une colonne PD10 (Amersham-Biosciences, UK) préalablement équilibrée en PBS pH 7, EDTA 5mM et éluée dans ce même tampon. Les fractions protéiques sont rassemblées selon les méthodes bien connues.

D'autre part, 2 ml de DMSO contenant 100 mg de SICC sont incubés avec 2 ml d'une solution de 50 mg/ml d'ampicilline sodique en tampon NaPi 25 mM, pH 8, pendant 60 minutes à 25°C, avant d'être incubé en présence de 50 mg de solution protéique pendant 4 heures à 4°C. La solution est enfin dialysée deux fois 6 heures contre 100 volumes de tampon NaPi 25 mM, pH 7,5.

### Préparation d'une solution d'avidine neutralisée

Une solution de 5 mg/ml d'avidine d'oeuf, disponible chez Pierce Inc, USA, est préparée en tampon NaPi 50 mM, pH 7,5.

### Préparation d'une solution contrôle de BSA-Or

La présente invention préconise l'utilisation d'une ligne de contrôle ayant une intensité constante et visible avant et après le développement du test. Cette ligne est de préférence de couleur similaire à la couleur développée aux lignes "tests" et est synthétisée comme suit.

A une préparation de 27 ml d'or colloïdal de 40 nm, obtenue selon la méthode de Frens ⁽⁸⁾ et dont la densité optique DO_{lamda max} est de 3, sont ajouté 3 ml d'une solution de BSA (Sigma) à 10% en tampon borate 10 mM, pH 6,5. Le mélange est incubé pendant 60 minutes à 20°C avant d'être centrifugé 45 minutes à 10.000 rpm dans un rotor SS34 (Sorvall). Le culot est ensuite récupéré dans du tampon NaPi 50 mM, NaCl 150 mM de manière à obtenir une DO_{lambda max} de 45. La solution qui sera déposée sur la membrane de nitrocellulose est encore diluée 15 fois dans le même tampon pour obtenir une DO finale de 3.

### Technique immunochromatographique

La technique immunochromatographique est connue et décrite dans la littérature (Developing Immunochromatographic Test Strips : A short Guide, Millipore, Lit. No. TB500 Printed in USA 11/96, 96-204). Dans la présente invention, les préparations obtenues ci-dessus sont déposées en des endroits précis et distincts sur une membrane en nitrocellulose et de préférence successivement l'un derrière l'autre en se référant au sens de migration du liquide. La membrane en nitrocellulose est ensuite placée en contact à une extrémité avec une membrane par exemple de type 142 disponible chez Ahlstrom, Inc., USA ou de type GFDVA disponible chez Whatman, UK, mais pas de type Leukosorb^{®} disponible chez Pall, UK et à l'autre extrémité par un papier absorbant quelconque par exemple de type 17CHR disponible chez Whatman, UK.

### Dépôt des éléments récupérateurs sur le support de nitrocellulose

C'est le positionnement des éléments récupérateurs qui permettront d'identifier le type de contamination rencontrée dans l'échantillon. Les solutions sont déposées à l'aide d'un "dispenseur" Biodot (UK) de type Quanti-3000, à un débit de 1 µL/cm.

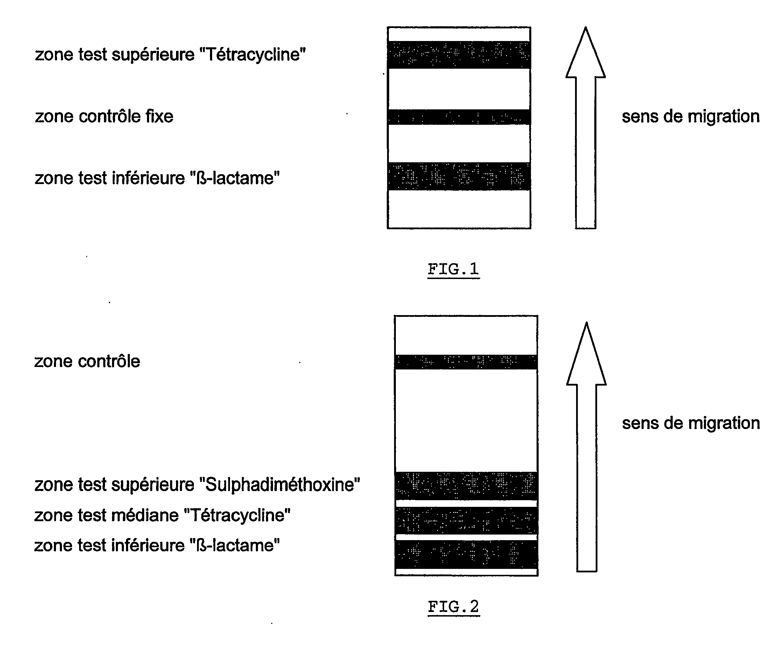

Dans le cas d'une détection de deux paramètres, c'est de part et d'autre de la ligne contrôle que les préparations de β-lactoglobuline et d'avidine seront déposées. Par exemple le conjugué β-lactoglobuline préparé ci avant, sera déposé en dessous de la ligne contrôle et la solution d'avidine neutralisée sera déposée au-dessus de la ligne de contrôle (figure 1). En conséquence, la présence de composé β-lactames se caractérisera par une absence de marquage sur la β-lactoglobuline, c'est-à-dire à la ligne test située en dessous de la ligne de contrôle et la présence de composés tétracyclines se caractérisera par une absence de marquage à la ligne test située au-dessus de la ligne contrôle. Dans le cas où les deux composés sont présents en quantité suffisante aucun des deux signaux n'apparaîtra, de part et d'autre de la ligne de contrôle (voir figure 1).

### Dosage simultané des β-lactames et des tétracyclines dans un échantillon de lait

200 µL de lait froid, sont incubés à 50°C en présence de 50 µl des réactifs préparés et/ou lyophilisés comme ci-dessus. Après 3 minutes d'incubation à 50°C, l'élément récupérateur décrit ci-dessus est plongée dans la solution. L'interprétation finale est faite après 3 minutes d'incubation à l'aide d'un lecteur optique de tigette disponible chez Matest (Allemagne).

Les résultats sont repris dans le tableau 1. Le procédé permet de considérer comme positif un échantillon de lait contenant 4 ppb en Ampicilline et/ou 75 ppb en Tétracycline.

Le tableau 2 résume les limites de détection obtenues par le présent procédé pour les divers composés de β-lactames et de tétracyclines renseignés. En règle générale lorsque le rapport des intensités du signal de test concerné par rapport au signal de contrôle est égal ou inférieur à 1, l'échantillon est considéré comme positif pour le paramètre (composé) concerné.

**Tableau 1 : Unités et rapports des intensités mesurées aux points de capture**

| ***Concentration antibiotique (ppb)*** | ***Intensité zone B (*)*** | ***Rapport B* / *C*** | ***Intensité zone T*** | ***Rapport T* / *C*** | ***Intensité zone contrôle*** |
|---|---|---|---|---|---|
| Ampi 0 + Tetra 0 | 520 | 2.5 | 410 | 2.0 | 208 |
| Ampi 1 | 380 | 1.9 | 380 | 1.9 | 199 |
| Ampi 2 | 250 | 1.2 | 405 | 1.9 | 211 |
| Ampi 3 | 155 | 0.8 | 408 | 2.0 | 205 |
| Ampi 4 | 105 | 0.5 | 386 | 1.9 | 201 |
| Ampi 5 | 55 | 0.3 | 397 | 2.0 | 197 |
| Ampi 10 | 25 | 0.1 | 414 | 2.1 | 200 |
| Ampi 0 + Tetra 25 | 505 | 2.5 | 340 | 1.7 | 206 |
| Ampi 0 + Tetra 50 | 521 | 2.5 | 288 | 1.4 | 206 |
| Ampi 0 + Tetra 75 | 523 | 2.6 | 162 | 0.8 | 203 |
| Ampi 0 + Tetra 100 | 508 | 2.6 | 55 | 0.3 | 199 |
| Ampi 4 + Tetra 75 | 122 | 0.6 | 165 | 0.8 | 202 |

| | | | | | |
|---|---|---|---|---|---|
| (*) B : beta ; T : tetra ; C : contrôle | | | | | |

**Tableau 2 : Limite de détection**

| ***Antibiotique*** | ***MRL*/*UE (ppb)*** | ***Safe Level*/*US (ppb)*** | ***Concentration (ppb)*** | ***Rapport d'intensité paramètre*/*zone contrôle*** |
|---|---|---|---|---|
| Benzylpénicilline | 4 | 5 | 2 | 0.4 |
| Ampicilline | 4 | 10 | 4 | 0.4 |
| Amoxicilline | 4 | 10 | 4 | 0.4 |
| Cloxacycline | 30 | 10 | 10 | 0.5 |
| Céphapyrine | 60 | 20 | 8 | 0.3 |
| Ceftiofur | 100 | 50 | 10 | 0.5 |
| Tétracycline | 100 | 300 | 75 | 0.8 |
| Oxytétracycline | 100 | 300 | 50 | 0.8 |
| Chlortétracycline | 100 | 300 | 50 | 0.8 |
| Doxycycline | 100 | 300 | 20 | 0.8 |

### Exemple N°2 : Dosage simultané des tétracyclines, β-lactames et sulfamides (format 1 pour les trois détections)

Dans cette forme d'exécution préférée, le procédé intègre en plus des deux récepteurs des tétracyclines et β-lactames, un anticorps spécifique pour la reconnaissance des sulfadiméthoxines.

### Préparation du mélange réactionnel

Au mélange renseigné ci-dessus, est intégré en outre un anticorps anti-sulphadiméthoxine selon la préparation suivante :
- 5 parts de récepteur β-lactame (RSA) à une concentration de 360 nM ;
- 5 parts de récepteur tétracycline (TetR) à une concentration de 4,2 µM ;
- 1 part de sérum anti-RSA diluée 4 x en tampon NaPi 50 mM, NaCl 150 mM, pH, 7,8 ;
- 1 part de sérum anti-TetR diluée 4 x dans ce même tampon ;
- 1 part de sérum anti-sulphadiméthoxine diluée 2 x dans ce même tampon ;
- 1 part d'acide nucléique à une concentration de 50 µM ;
- 20 parts de protéine-A Or de 40 nm à une DO=10 à 520nm ;
- 16 parts de tampon Hepes 120 mM, pH 8, BSA 2%, Dextran 1%, sucrose 5%.
Ce mélange est soit préparé extemporanément soit lyophilisé pendant 20 heures.

### Le système récupérateur

Le système récupérateur est semblable à celui de l'exemple 1, dans lequel une quatrième ligne spécifique pour la récupération des molécules d'anticorps anti-sulphadiméthoxine est intégrée (3^{ème} ligne de test). Les autres zones de capture sont identiques mais positionnées autrement selon la figure 2.

### Préparation d'une BSA conjuguée à une sulphadiméthoxine

La préparation est réalisée en suivant le protocole décrit par Dixon-Holland et Katz⁽⁹⁾. 100 mg de sulfadiméthoxine et 200 mg de BSA solubilisés dans 25 ml d'un mélange, contenant 2 parts de tampon phosphate 50 mM pH 7,2 et 1 part de dioxane, sont incubés en présence de 120 µL de 25% glutaraldéhyde sous agitation pendant 3 heures à 25°C. La solution est ensuite dialysée pendant 6 jours à 4°C contre 100 volumes de tampon NaPi 50 mM, pH 7,2 avec changement de tampon toutes les 12 heures.

### Dépôt des éléments récupérateurs sur le support de nitrocellulose

Dans le cas d'une détection de trois paramètres, les lignes de capture sont disposées en des endroits précis et distincts sur une membrane en nitrocellulose et de préférence successivement l'une dernière l'autre en se référant au sens de migration du liquide. Selon une modalité préférée, les zones de capture β-lactame, tétracycline, sulphadiméthoxine et la zone contrôle sont disposées respectivement à un premier, à un deuxième, à un troisième et enfin à un quatrième niveau en se référant au sens de migration du liquide (figure 2). Une seule zone de contrôle sert de référence pour les trois marqueurs. Une formulation différente, similaire à l'exemple précédent, serait d'intégrer deux zones de contrôle, c'est-à-dire une zone de contrôle entre chacune des zones de test.

On remarquera de manière générale que, selon la présente invention, les lignes de test et de contrôle ne doivent pas être toujours obligatoirement disposées dans le même ordre par rapport au sens de migration du liquide. Ainsi la trousse fonctionne aussi bien si l'on a respectivement l'ordre « beta » -« tetra » ou bien « tetra » - « beta », « beta » - « sulfa » ou bien « sulfa » - « beta » (voir exemple ci-après), « beta » - « tetra » - « sulfa » ou bien « sulfa » - « tetra » - « beta », etc.

### Dosage simultané des ß-lactames, des tétracyclines et des sulphadiméthoxines dans un échantillon de lait

200 µL de lait froid sont incubés à 50°C en présence de 50 µl des réactifs préparés et/ou lyophilisés comme ci dessus. Après 3 minutes d'incubation à 50°C, l'élément récupérateur décrit ci-dessus est plongée dans la solution. L'interprétation finale est faite après 3 minutes d'incubation à l'aide d'un lecteur optique de tigette disponible chez Matest (Allemagne).

Les résultats sont repris dans le tableau 3. Le procédé permet de considérer positif un échantillon de lait contenant 4 ppb en ampicilline et/ou 75 ppb en tétracycline et 100 ppb en sulphadiméthoxine.

Le tableau 4 résume les limites de détection obtenues par le procédé de l'invention pour les divers composés de β-lactames, de tétracyclines et de sulphadiméthoxines renseignés. En règle générale, lorsque le rapport des intensités du signal de test concerné par rapport au signal de contrôle est égal ou inférieur à 1, l'échantillon est considéré comme positif pour le paramètre (composé) concerné.

**Tableau 3 : Rapports des intensités mesurées**

| ***Concentration antibiotique (ppb)*** | | | ***Rapport des intensités de mesure*** | | |
|---|---|---|---|---|---|
| ***A (*)*** | ***T*** | ***S*** | ***B*/*C*** | ***T*/*C*** | ***S*/*C*** |
| 0 | 0 | 0 | 2.5 | 2.0 | 1.9 |
| 1 | 0 | 0 | 1.9 | 1.9 | 1.7 |
| 2 | 0 | 0 | 1.2 | 1.9 | 2.1 |
| 3 | 0 | 0 | ***0.8*** | 2.0 | 2.2 |
| ***4*** | 0 | 0 | ***0.5*** | 1.9 | 2.0 |
| ***5*** | 0 | 0 | ***0.3*** | 2.0 | 2.0 |
| **10** | 0 | 0 | ***0.1*** | 2.1 | 1.9 |
| 0 | ***25*** | 0 | 2.5 | ***1.7*** | 1.9 |
| 0 | ***50*** | 0 | 2.5 | ***1.4*** | 2.2 |
| 0 | ***75*** | 0 | 2.6 | ***0.8*** | 2.0 |
| 0 | ***100*** | 0 | 2.6 | ***0.3*** | 1.8 |
| 0 | 0 | 50 | 2.4 | 1.9 | 1.3 |
| 0 | 0 | ***100*** | 2.2 | 1.9 | ***0.8*** |
| 0 | 0 | ***150*** | 1.9 | 2.1 | ***0.6*** |
| 0 | 0 | ***200*** | 2.4 | 2.1 | ***0.4*** |
| 4 | 75 | ***100*** | ***0.6*** | ***0.8*** | ***0.7*** |

| | | | | | |
|---|---|---|---|---|---|
| (*) A : ampicilline ; T : tétracycline ; S : sulfamide ; C : contrôle | | | | | |

**Tableau 4 : Limite de détection**

| ***Antibiotique*** | ***Concentration limite détection (ppb)*** | ***Rapport Test*/*Contrôle*** |
|---|---|---|
| Benzylpenicilline | 2 | 0.4 |
| Ampicilline | 4 | 0.4 |
| Amoxicilline | 4 | 0.4 |
| Cloxacycline | 10 | 0.5 |
| Céphapyrine | 8 | 0.3 |
| Ceftiofur | 10 | 0.5 |
| Tétracycline | 75 | 0.8 |
| Oxytétracycline | 50 | 0.9 |
| Chlortétracycline | 50 | 0.7 |
| Doxycycline | 20 | 0.6 |
| Sulphadimétoxine | 100 | 0.8 |

### Exemple N°3 : Dosage simultané des β-lactames et des sulfamides (format 1 pour les β-lactames et format 2 pour les sulfamides)

### Préparation du mélange réactionnel

Le mélange réactionnel comprend :
- 5 parts de récepteur ß-lactame (RSA) à une concentration de 360 nM ;
- 10 parts d'un anticorps monoclonal anti-RSA couplé à de l'or colloïdal (DO=10 à 520 nm) ;
- 1 part d'anti-sulphadiméthoxine biotinylé et dilué en NaPi 50 mM, NaCl 150 mM, pH 7,8 ;
- 10 parts d'une dilution de BSA-sulphadiméthoxine-Or de 40 nm ;
- 24 parts de tampon Hepes 120 mM, pH 8, BSA 2%, Dextran 1%, sucrose 5%.
Ce mélange est soit préparé extemporanément, soit lyophilisé pendant 20 heures.

### Biotinylation de l'anticorps anti-sulphadiméthoxine

825 µL d'une solution contenant 5 mg/ml d'anticorps anti-sulphadiméthoxine dialysé en tampon carbonate 100 mM, pH 9,2 sont incubés en présence de 165 µL d'une solution de Biotine-LC-NHS à 1,5 mg/ml (disponible chez Perbio, Inc) pendant 2 heures à 25°C à l'abri de la lumière. La réaction est bloquée par ajout de 10 µL d'une solution de tampon tris 1M pH 8 pendant 30 minutes avant dialyse 2 fois 10 heures contre du tampon NaPi 50 mM, NaCl 150 mM, pH 7,8.

### Préparation d'une BSA-sulphadiméthoxine conjuguée à des particules d'or

La préparation de BSA-sulphadiméthoxine décrite à l'exemple n°2 est utilisée en couplage sur des particules d'or colloïdal dans un protocole similaire à celui décrit dans l'exemple n°1 pour préparer la solution contrôle BSA-Or.

### Le système récupérateur

Le système récupérateur est identique à celui de l'exemple 1. Cependant dans le cas présent, c'est l'anticorps anti-sulfamide biotinylé qui sera récupéré dans l'avidine immobilisée en deuxième point de capture au-dessus de la zone contrôle.

### Dosage simultané des ß-lactames et des sulphadiméthoxines dans un échantillon de lait

200 µL de lait froid sont incubés pendant 15 minutes à 30°C en présence de 50 µL des réactifs préparés ci-dessus. Après cette première incubation, l'élément récupérateur décrit ci-dessus est plongé dans la solution. L'interprétation finale est faite après une seconde incubation de 15 minutes. Les résultats sont repris dans le tableau 5.

**Tableau 5: Rapports des intensités mesurées**

| ***Concentration antibiotique (ppb)*** | | ***Rapport des* intensités de mesure** | |
|---|---|---|---|
| ***B(*)*** | ***S*** | ***B*/*C*** | ***S*/*C*** |
| 0 | 0 | 2 | 3 |
| 1 | 0 | 1.5 | 2.8 |
| 2 | 0 | 1.2 | 2.9 |
| **3** | 0 | ***0.95*** | 3 |
| ***4*** | 0 | ***0.8*** | 3.1 |
| ***5*** | 0 | ***0.65*** | 3.1 |
| ***10*** | 0 | ***0.35*** | 2.9 |
| 0 | 0 | 2 | 2.9 |
| 0 | 50 | 1.95 | 2 |
| 0 | ***100*** | 1.9 | ***1*** |
| 0 | ***150*** | 2.2 | ***0.85*** |
| 0 | ***200*** | 2.1 | ***0.7*** |
| ***4*** | ***100*** | ***0. 84*** | ***0.95*** |

| | | | |
|---|---|---|---|
| (*) B : β-lactame ; S : sulfamide ; C : contrôle | | | |

### Exemple N°4 : Dosage simultané des tétracyclines et des sulfamides (format 2 pour les deux détections)

### Préparation du mélange réactionnel

Le mélange réactionnel comprend :
- 5 parts de récepteur tétracycline (TetR) à une concentration de 4,2 µM ;
- 1 part d'anticorps monoclonal de souris anti-TetR dilué en tampon NaPi 50 mM, NaCl 150 mM, pH 7,8 ;
- 1 part d'anticorps de lapin anti-sulfamide dilué dans ce même tampon ;
- 10 parts d'une dilution de BSA-sulphadiméthoxine-Or de 40 nm ;
- 1 part d'acide nucléique biotinylé à une concentration de 50 µM ;
- 10 parts d'un anticorps anti-biotine-Or de 40 nm à une DO=10 à 520nm ;
- 22 parts de tampon Hepes 120 mM, pH 8, BSA 2%, Dextran 1%, sucrose 5%.
Ce mélange est soit préparé extemporanément, soit lyophilisé pendant 20 heures.

### Le système récupérateur

Le système récupérateur est similaire à celui de l'exemple 1. Cependant dans le cas présent, c'est un anticorps anti-souris qui constitue le premier point de capture où sera récupéré l'anticorps monoclonal anti-TetR et c'est un anticorps de poulet anti-lapin qui constitue le second point de capture où sera récupéré l'anticorps anti-sulfamide.

### Dosage simultané des tétracyclines et des sulfamides dans un échantillon de viande

A 10 g de muscle de porc sont ajoutés 30 ml de tampon NaPi 50 mM, pH 8 et le mélange est malaxé à l'aide d'un malaxeur de type MiniMix (Interscience, F) pendant 2 minutes. La solution, reprise dans un tube Eppendorf, est ensuite centrifugée 1 minute à 6000 rpm pour en récupérer le surnageant. 200 µL de surnageant sont incubés pendant 15 minutes à 25°C en présence de 50 µL des réactifs préparés ci-dessus. Après cette première incubation, l'élément récupérateur décrit ci-dessus est plongé dans la solution. L'interprétation finale est faite après une seconde incubation de 15 minutes. Les résultats sont repris dans le tableau 6.

**Tableau 6 : Rapports des intensités mesurées**

| ***Concentration antibiotique (ppb)*** | | ***Rapport des intensités de mesure*** | |
|---|---|---|---|
| ***T(*)*** | ***S*** | ***T*/*C*** | ***S*/*C*** |
| 0 | 0 | 3.2 | 2.1 |
| 25 | 0 | 2.5 | 2.2 |
| 50 | 0 | 1.6 | 2.2 |
| ***75*** | 0 | ***1.1*** | 2.1 |
| ***100*** | 0 | ***0.5*** | 2 |
| ***200*** | 0 | ***0*** | 1.9 |
| 0 | 0 | 3.2 | 2.2 |
| 0 | 50 | 3.4 | 1.6 |
| 0 | ***100*** | 3.1 | ***1*** |
| 0 | ***150*** | 3.4 | ***0.85*** |
| 0 | ***200*** | 3 | ***0.7*** |
| ***100*** | ***100*** | ***0.4*** | ***0.95*** |

| | | | |
|---|---|---|---|
| (*) T : tétracycline ; *S* : sulfamide ; C : contrôle | | | |

### Exemple 5 : Dosage simultané des β-lactames, des tétracyclines et des sulfamides (format 1 pour les β-lactames/tétracyclines et format 2 pour les sulfamides)

### Préparation du mélange réactionnel

Le mélange réactionnel comprend :
- 5 parts de récepteur ß-lactame (RSA) à une concentration de 360 nM ;
- 1 part d'un anticorps monoclonal de souris anti-RSA diluée en tampon NaPi 50 mM, NaCl 150 mM, pH 7,8 ;
- 5 parts de récepteur tétracycline (TetR) à une concentration de 4,2 µM ;
- 1 part d'un anticorps monoclonal de souris anti-TetR diluée en tampon NaPi 50 mM, NaCl 150 mM, pH 7,8 ;
- 1 part d'acide nucléique biotinylé à une concentration de 50 µM ;
- 1 part d'anticorps polyclonal de lapin anti-sulfamide dilué en tampon NaPi 50 mM, NaCl 150 mM, pH 7,8 ;
- 7 parts d'une dilution de BSA-sulphadiméthoxine-Or de 40 nm ;
- 13 parts d'un anticorps anti-souris couplé à de l'or colloïdal de 40 nm à une DO=10 à 520nm ;
- 16 parts de tampon Hepes 120 mM, pH 8, BSA 2%, Dextran 1%, sucrose 5%.
Ce mélange est soit préparé extemporanément, soit lyophilisé pendant 20 heures.

Dans une seconde méthode préférée, les deux anticorps monoclonaux anti-RSA et anti-TetR sont directement conjugués à des particules d'or colloïdal.

### Le système récupérateur

Le système récupérateur est similaire à celui de l'exemple 1. Cependant, dans le cas présent, c'est la lactoglobuline-ampicilline qui constitue le premier point de capture où sera récupéré le récepteur RSA, c'est de l'avidine en deuxième point de capture où sera récupéré le récepteur TetR et c'est un anticorps de poulet anti-lapin qui constitue le troisième point de capture où sera récupéré l'anticorps de lapin anti-sulfamide.

### Dosage simultané des β-lactames, des tétracyclines et des sulfamides dans un échantillon de lait

200 µL de lait froid sont incubés pendant 15 minutes à 30°C en présence de 50 µL des réactifs préparés ci-dessus. Après cette première incubation, l'élément récupérateur décrit ci-dessus est plongé dans la solution. L'interprétation finale est faite après une seconde incubation de 15 minutes. Les résultats sont repris dans le tableau 7.

**Tableau 7 : Rapports des intensités mesurées**

| ***Concentration antibiotique (ppb)*** | | | ***Rapport des intensités de mesure*** | | |
|---|---|---|---|---|---|
| ***B* (*)** | ***T*** | ***S*** | ***B*/*C*** | ***T*/*C*** | ***S*/*C*** |
| 0 | 0 | 0 | 2 | 3 | 1.8 |
| 2 | 0 | 0 | 1.3 | 2.7 | 1.8 |
| ***4*** | 0 | 0 | ***0.9*** | 2.7 | 1.7 |
| ***10*** | 0 | 0 | ***0.5*** | 2.8 | 1.9 |
| 0 | 50 | 0 | 1.9 | ***1.4*** | 1.9 |
| 0 | ***100*** | 0 | 2 | ***0.6*** | 2 |
| 0 | 0 | 50 | 2.1 | 2.8 | 1.3 |
| 0 | 0 | ***100*** | 1.8 | 2.7 | ***0.9*** |
| 0 | 0 | ***200*** | 1.8 | 3 | ***0.3*** |
| ***4*** | ***100*** | ***100*** | ***0.95*** | ***0.7*** | ***0.8*** |

| | | | | | |
|---|---|---|---|---|---|
| (*) B :β-lactame ; T : tétracycline ; S : sulfamide ; C : contrôle | | | | | |

### Références

⁽¹⁾ DIRECTIVE 96/23/CE DU CONSEIL, du 29 avril 1996, *relative aux mesures de contrôle à* mettre *en oeuvre à l'égard de certaines substances et leurs résidus dans les animaux vivants et leurs produits et abrogeant les Directives 85*/*358*/*CEE et 86*/*469*/*CEE et les Décisions 89*/*187*/*CEE et 91*/*664*/*CEE.*
⁽²⁾REGLEMENT (CEE) n° 2377/90 DU CONSEIL, du 26 juin 1990, *établissant une procédure communautaire pour la fixation des limites maximales* de *résidus* de *médicaments vétérinaires dans les aliments d'origine animale.*
⁽³⁾ Off. J. Eur. Comm. 2002, L221/8 - 2002/657/CE.
⁽⁴⁾ Hisao Oka, Chemical Analysis for antibiotics Used in Agriculture, by AOAC INTERNATIONAL, 1995, ISBN 0-935584-57-9.
⁽⁵⁾ Ron Verheijen et al, Analyst 123 (1998), 2437-2441.
⁽⁶⁾ Golemi-Kotra, D. et al, The Journal of Biological Chemistry, Vol. 278, n° 20 (May 2003), pp. 18419-18425.
⁽⁷⁾ Kachab, E.H. et al, The Journal of Immunology Methods, Vol 147, n°1 (1992), pp. 33-41.
⁽⁸⁾ Frens, G., Nature (London), Phys. Sci., 241 (1973), 20.
⁽⁹⁾ Dixon-Holland, D.E. et Katz, S.E., (1988), J. Assoc. Off. Anal. Chem. (1988) 71 (6), 1137-40.

## Revendications

1. Trousse de diagnostic pour le dosage simultané d'antibiotiques de classes différentes, au moins des β-lactames et des tétracyclines, **caractérisée en ce qu'**elle comprend :
- d'une part, un seul mélange réactionnel contenant au moins un premier récepteur, spécifique de la reconnaissance des β-lactames, un second récepteur, reconnaissant de manière spécifique et compétitive une tétracycline et un fragment d'acide nucléique biotinylé, les deux récepteurs étant marqués de préférence par des particules d'or colloïdal, soit directement, soit indirectement par l'intermédiaire d'un anticorps ou d'un anticorps en association avec de la protéine-A ;
- et d'autre part, un système récupérateur sous la forme d'un support solide comprenant une membrane de nitrocellulose sur laquelle sont fixés en deux endroits connus et distincts appelés zones de test, respectivement un antibiotique à noyau β-lactame et une avidiné ou inversement ;
de sorte que l'intensité de marquage détectée sur le système récupérateur aux deux zones de test résulte de manière indépendante d'une reconnaissance compétitive de chaque antibiotique par son récepteur marqué.

2. Trousse de diagnostic selon la revendication 1, **caractérisée en ce que** l'antibiotique à noyau ß-lactame et l'avidine fixés sur la membrane de nitrocellulose sont respectivement une pénicilline ou une céphalosporine, de préférence une ampicilline immobilisée sur une β-lactoglobuline, et une avidine d'oeuf.

3. Trousse de diagnostic pour le dosage simultané d'antibiotiques de classes différentes, au moins des β-lactames et des sulfamides, **caractérisée en ce qu'**elle comprend :
- d'une part, un seul mélange réactionnel contenant au moins un premier récepteur, spécifique de la reconnaissance des β-lactames, marqué soit directement soit indirectement de préférence par des particules d'or colloïdal, un anticorps antisulfamide biotinylé et un analogue d'un sulfamide marqué soit directement soit indirectement à l'or colloïdal ;
- et d'autre part, un système récupérateur sous la forme d'un support solide comprenant une membrane de nitrocellulose sur laquelle sont fixés en deux endroits connus et distincts, appelés zones de test, respectivement un antibiotique à noyau β-lactame et une avidine ou inversement ;
de sorte que l'intensité de marquage détectée sur le système récupérateur aux deux zones de test résulte de manière indépendante d'une reconnaissance compétitive de chaque antibiotique.

4. Trousse de diagnostic pour le dosage simultané d'analytes correspondant à des antibiotiques de classes différentes, au moins des tétracyclines et des sulfamides, **caractérisée en ce qu'**elle comprend :
- d'une part, un seul mélange réactionnel contenant au moins un récepteur, reconnaissant de manière spécifique et compétitive une tétracycline et un fragment d'acide nucléique biotinylé, ledit récepteur étant marqué de préférence par des particules d'or colloïdal, soit directement, soit indirectement par l'intermédiaire d'un anticorps, ainsi qu'un anticorps antisulfamide et un analogue d'un sulfamide marqué soit directement soit indirectement de préférence à l'or colloïdal ;
- et d'autre part, un système récupérateur sous la forme d'un support solide comprenant une membrane de nitrocellulose sur laquelle sont fixés en deux endroits connus et distincts, appelés zones de test, respectivement un anticorps dirigé spécifiquement soit contre le récepteur de tétracycline, soit contre l'anticorps spécifique du récepteur de tétracycline et un anticorps dirigé spécifiquement contre l'anticorps antisulfamide ou inversement.

5. Trousse de diagnostic selon l'une quelconque des revendications 1 à 4, **caractérisée en ce que** les anticorps anti-récepteurs spécifiques ajoutés au mélange réactionnel sont des anticorps soit monoclonaux, soit polyclonaux, modifiés ou non modifiés chimiquement ou par recombinaison, purifiés ou non purifiés, couplés ou non couplés, directement ou indirectement, à des particules marquées, de préférence des particules d'or colloïdal.

6. Trousse de diagnostic selon l'une quelconque des revendications 1 à 5, **caractérisée en ce que** la membrane de nitrocellulose, déposée sur un support tigette, est en contact à une première extrémité avec une membrane et à une seconde extrémité avec un papier absorbant et présente les deux zones de test distinctes l'une derrière l'autre dans le sens de migration du liquide, une zone de contrôle pouvant éventuellement séparer les deux zones de test.

7. Trousse de diagnostic selon la revendication 6, **caractérisée en ce que** la zone de contrôle est obtenue à partir d'une préparation quelconque de protéine marquée, de préférence par des particules d'or colloïdal et de manière préférée encore à partir d'une sérumalbumine bovine ou BSA couplée à des particules d'or colloïdal.

8. Trousse de diagnostic pour le dosage simultané de β-lactames, de tétracyclines et de sulfamides, selon la revendications 1 ou 2, **caractérisé en ce que** le mélange réactionnel comprend en outre un anticorps spécifique de la reconnaissance des sulfamides et marqué, de préférence directement ou indirectement par des particules d'or colloïdal et **en ce que** le système récupérateur comprend en outre une préparation de protéine conjuguée à des sulfamides, immobilisée sur la membrane de nitrocellulose.

9. Trousse de diagnostic pour le dosage simultané de β-lactames, de tétracyclines et de sulfamides, selon la revendication 8, **caractérisée en ce que** la membrane de nitrocellulose, déposée sur un support tigette, est en contact à une première extrémité avec une membrane et à une seconde extrémité avec un papier absorbant et présente trois zones de test distinctes et une zone de contrôle, disposées successivement l'une derrière l'autre dans le sens de migration du liquide, ou deux zones de contrôle, chacune disposée alternativement entre deux zones de test.

10. Trousse de diagnostic pour le dosage simultané d'analytes correspondant à des antibiotiques de classes différentes, au moins des β-lactames, des tétracyclines et des sulfamides, **caractérisée en ce qu'**elle comprend :
- d'une part, un seul mélange réactionnel contenant au moins un premier récepteur, spécifique de la reconnaissance des β-lactames, un second récepteur, reconnaissant de manière spécifique et compétitive une tétracycline et un fragment d'acide nucléique biotinylé, les deux récepteurs étant marqués de préférence par des particules d'or colloïdal, soit directement, soit indirectement par l'intermédiaire d'un anticorps ou d'un anticorps en association avec de la protéine-A, ainsi qu'un anticorps antisulfamide ainsi qu'un analogue d'un sulfamide libre marqué soit directement soit indirectement à l'or colloïdal;
- et d'autre part, un système récupérateur sous la forme d'un support solide comprenant une membrane de nitrocellulose sur laquelle sont fixés en trois endroits connus et distincts, respectivement un antibiotique à noyaux ß-lactame, une avidine et un anticorps dirigé contre l'anticorps antisulfamide,
de sorte que l'intensité de marquage détectée sur le système récupérateur aux trois zones de test résulte de manière indépendante d'une reconnaissance compétitive de chaque antibiotique.

11. Trousse de diagnostic selon l'une quelconque des revendications précédentes, **caractérisé en ce que** l'échantillon est essentiellement liquide et est obtenu à partir de lait, de miel, de viande, d'oeufs ou de liquides biologiques.

12. Trousse de diagnostic selon l'une quelconque des revendications précédentes, **caractérisée en ce que** les récepteurs utilisés pour le dosage des ß-lactames ou des tétracyclines sont respectivement les récepteurs BlaR et TetR isolés à partir de classes connues de microorganismes, de préférence respectivement à partir de *Staphylococcus aureus* pour BlaR et du plasmide pSC101 *d'E. coli 600* pour TetR.

13. Procédé de mise en oeuvre d'une trousse de diagnostic selon l'une quelconque des revendications précédentes, **caractérisé par** les étapes suivantes :
- on met en contact le mélange réactionnel précité avec un échantillon à caractériser pour obtenir une solution qu'on laisse incuber à une température comprise entre 30°C et 50°C pendant 3 à 15 minutes ;
- on plonge une tigette portant le système récupérateur précité dans la solution obtenue et on laisse incuber pendant 3 à 15 minutes ;
- on interprète le résultat sur la tigette soit visuellement à l'oeil, soit au moyen d'un lecteur optique de tigette.

## Claims

1. Diagnostic kit for the simultaneous measurement of antibiotics of different classes, at least β-lactames and tetracyclines, **characterised in that** it comprises:
- for one thing, a single reactive mixture comprising at least a first receptor specific for the recognition of β-lactames, a second receptor to specifically and competitively recognise a tetracycline and a biotinylated fragment of nucleic acid, both receptors preferably being marked by particles of colloidal gold, either directly or indirectly by means of an antibody or of an antibody in association with protein A;
- and for another thing, a recovery system in the form of a solid support comprising a nitrocellulose membrane on which are attached at two known and distinct sections, called test zones, an antibiotic with a β-lactame core and an avidine, respectively or vice versa;
in such a way that the marking intensity detected on the recovery system in both test zones independently results from the competitive recognition of each antibiotic by its marked receptor.

2. Diagnostic kit according to claim 1, **characterised in that** the antibiotic with a β-lactame core and the avidine bound to the nitrocellulose membrane are a penicillin or a cephalosporine respectively, preferably an ampicillin fixed to a β-lactoglobulin and an egg-white avidine.

3. Diagnostic kit for the simultaneous measurement of antibiotics of different classes, at least β-lactames and sulfamides, **characterised in that** it comprises:
- for one thing, a single reactive mixture comprising at least a first receptor specific for the recognition of β-lactames, marked directly or indirectly preferably by particles of colloidal gold, a biotinylated antisulfamide antibody and an analogue of a sulfamide marked either directly or indirectly with colloidal gold;
- and for another thing, a recovery system in the form of a solid support comprising a nitrocellulose membrane on which are attached at two known and distinct sections, called test zones, an antibiotic with a β-lactame core and an avidine, respectively or vice versa;
in such a way that the marking intensity detected in the recovery system in both test zones independently results from the competitive recognition of each antibiotic.

4. Diagnostic kit for the simultaneous measurement of analytes corresponding to antibiotics of different classes, at least tetracyclines and sulfamides, **characterised in that** it comprises:
- for one thing, a single reactive mixture comprising at least one receptor that specifically and competitively recognises a tetracycline and a biotinylated fragment of nucleic acid, said receptor being preferably marked by particles of colloidal gold either directly or indirectly by means of an antibody, as well as an antisulfamide antibody and an analogue of a sulfamide marked either directly or indirectly preferably with colloidal gold;
- and for another thing, a recovery system in the form of a solid support comprising a nitrocellulose membrane to which are attached at two known and distinct sections, called test zones, an antibody specifically targeting either the tetracycline receptor or the specific antibody of the tetracycline receptor and an antibody specifically targeting the antisulfamide antibody, respectively or vice versa.

5. Diagnostic kit according to any one of Claims 1 to 4, **characterised in that** the specific anti-receptor antibodies added to the reactive mixture are monoclonal or polyclonal antibodies, modified or unmodified chemically or by recombination, purified or unpurified, attached or unattached, directly or indirectly to marked particles, preferably particles of colloidal gold.

6. Diagnostic kit according to any one of Claims 1 to 5, **characterised in that** the nitrocellulose membrane deposited on a dipstick support is in contact at a first end with a membrane and at a second end with an absorbent paper and has both distinct test zones one after the other in the migration direction of the liquid, a control zone possibly separating the two test zones.

7. Diagnostic kit according to Claim 6, **characterised in that** the control zone is obtained by means of any protein preparation marked preferably by particles of colloidal gold and still preferably by means of a bovine serumalbumin or BSA attached to particles of colloidal gold.

8. Diagnostic kit for the simultaneous measurement of β-lactames, tetracyclines and sulfamides according to Claim 1 or 2, **characterised in that** the reactive mixture also comprises a antibody specific for the recognition of sulfamides and marked, preferably directly or indirectly by particles of colloidal gold, and **in that** the recovery system also comprises a preparation of protein conjugated with sulfamides, fixed to the nitrocellulose membrane.

9. Diagnostic kit for the simultaneous measurement of β-lactames, tetracyclines and sulfamides according to Claim 8, **characterised in that** the nitrocellulose membrane deposited on a dipstick support is in contact at a first end with a membrane and at a second end with an absorbent paper and has three distinct test zones and a control zone, successively arranged one after the other in the migration direction of the liquid, or two control zones, each alternately arranged between two test zones.

10. Diagnostic kit for the simultaneous measurement of analytes corresponding to antibiotics of different classes, at least β-lactames, tetracyclines and sulfamides, **characterised in that** it comprises:
- for one thing, a single reactive mixture comprising at least a first receptor specific for the recognition of β-lactames, a second receptor that specifically and competitively recognises a tetracycline and a biotinylated fragment of nucleic acid, both receptors being preferably marked by colloidal gold particles, either directly or indirectly by means of an antibody or an antibody in association with protein A, as well as an antisulfamide antibody and an analogue of a free sulfamide marked either directly or indirectly with colloidal gold;
- and for another thing, a recovery system in the form of a solid support comprising a nitrocellulose membrane to which are attached at three known and distinct sections an antibiotic with a β-lactame core, an avidine and an antibody targeting the antisulfamide antibody, respectively,
in such a way that the marking intensity detected in the recovery system at the three test zones independently results from the competitive recognition of each antibiotic.

11. Diagnostic kit according to any one of the preceding claims, **characterised in that** the sample is essentially liquid and is obtained from milk, honey, meat, eggs or biological liquids.

12. Diagnostic kit according to any one of the preceding claims, **characterised in that** the receptors used for the measurement of the β-lactames or tetracyclines are the receptors BlaR and TetR respectively isolated from known classes of micro-organisms, preferably from *Staphylococcus* aureus for BlaR and from plasmid pSC101 of *E. coli 600* for TetR, respectively.

13. Method for implementing a diagnostic kit according to any one of the preceding claims, **characterised by** the following stages:
- the above-mentioned reactive mixture is brought into contact with a sample to be classified in order to obtain a solution that is left to incubate at a temperature between 30°C and 50°C for 3 to 15 minutes;
- a dipstick bearing the above-mentioned recovery system is immersed in the solution obtained and is left to incubate for 3 to 15 minutes;
- the result on the dipstick is interpreted either visually by the naked eye or by means of an optical dipstick reader.

## Patentansprüche

1. Diagnosekit für die gleichzeitige quantitative Bestimmung von Antibiotika aus verschiedenen Klassen, zumindest aus den β-Lactamen und den Tetracyclinen, **dadurch gekennzeichnet, dass** es Folgendes umfasst:
- einerseits eine einzige Reaktionsmischung, enthaltend mindestens einen ersten Rezeptor, der für das Erkennen von β-Lactamen spezifisch ist, einen zweiten Rezeptor, der auf spezifische und kompetitive Weise ein Tetracyclin und ein biotinyliertes Nucleinsäurefragment erkennt, wobei die beiden Rezeptoren vorzugsweise mittels kolloidaler Goldpartikel, entweder direkt oder indirekt mittels eines Antikörpers oder mittels eines an das Protein A gebundenen Antikörpers, markiert sind,
- und andererseits ein Auffangsystem in Form eines festen Trägers, umfassend eine Nitrocellulose-Membran, auf der an zwei bekannten und getrennten Stellen, die Testzonen genannt werden, jeweils ein Antibiotikum mit β-Lactamkern und ein Avidin gebunden sind oder umgekehrt;
so dass die auf dem Auffangsystem mit zwei Testzonen nachgewiesene Markierungsintensität auf unabhängige Weise aus einer kompetitiven Erkennung von jedem Antibiotikum durch seinen markierten Rezeptor resultiert.

2. Diagnosekit nach Anspruch 1, **dadurch gekennzeichnet, dass** das Antibiotikum mit einem β-Lactamkern und das Avidin, die auf der Nitrocellulose-Membran fixiert sind, jeweils ein Penicillin oder ein Cephalosporin, vorzugsweise ein auf einem β-Lactoglobulin immobilisiertes Ampicillin, und ein Avidin aus Ei sind.

3. Diagnosekit für die gleichzeitige quantitative Bestimmung von Antibiotika aus verschiedenen Klassen, zumindest aus den β-Lactamen und den Sulfamiden, **dadurch gekennzeichnet, dass** es Folgendes umfasst:
- einerseits eine einzige Reaktionsmischung, enthaltend mindestens einen Rezeptor, der für das Erkennen von β-Lactamen spezifisch ist und entweder direkt oder indirekt vorzugsweise mit kolloidalen Goldpartikel markiert ist, einen biotinylierten Antisulfamid-Antikörper und ein Sulfamid-Analogon, das entweder direkt oder indirekt mit kolloidalem Gold markiert ist;
- und andererseits ein Auffangsystem in Form eines festen Trägers, umfassend eine Nitrocellulose-Membran, auf der an zwei bekannten und getrennten Stellen, die Testzonen genannt werden, jeweils ein Antibiotikum mit β-Lactamkern und ein Avidin gebunden sind oder umgekehrt;
so dass die auf dem Auffangsystem mit zwei Testzonen nachgewiesene Markierungsintensität auf unabhängige Weise aus einer kompetitiven Erkennung von jedem Antibiotikum resultiert.

4. Diagnosekit für die gleichzeitige quantitative Bestimmung von Analyten, die Antibiotika aus verschiedenen Klassen, zumindest aus den Tetracyclinen und den Sulfamiden, entsprechen, **dadurch gekennzeichnet, dass** es Folgendes umfasst:
- einerseits eine einzige Reaktionsmischung, enthaltend mindestens einen Rezeptor, der auf spezifische und kompetitive Weise ein Tetracyclin und ein biotinyliertes Nucleinsäurefragment erkennt, wobei dieser Rezeptor vorzugsweise mittels kolloidaler Goldpartikel, entweder direkt oder indirekt mittels eines Antikörpers, markiert ist, sowie einen Antisulfamid-Antikörper und ein Sulfamid-Analogon, das entweder direkt oder indirekt vorzugsweise mit kolloidalem Gold markiert ist,
- und andererseits ein Auffangsystem in Form eines festen Trägers, umfassend eine Nitrocellulose-Membran, auf der an zwei bekannten und getrennten Stellen, die Testzonen genannt werden, jeweils ein Antikörper, der entweder spezifisch gegen den Tetracyclinrezeptor oder gegen den für den Tetracyclinrezeptor spezifischen Antikörper gerichtet ist, und ein Antikörper gebunden ist, der spezifisch gegen den Antisulfamid-Antikörper gerichtet ist, oder umgekehrt.

5. Diagnosekit nach einem der Ansprüche 1 bis 4, **dadurch gekennzeichnet, dass** die antirezeptorspezifischen Antikörper, die der Reaktionsmischung zugegeben werden, entweder monoklonale oder polyklonale, chemisch oder mittels Rekombination modifizierte oder nicht modifizierte, gereinigte oder ungereinigte Antikörper sind, die direkt oder indirekt an markierte Partikel, vorzugsweise kolloidale Goldpartikel, gekoppelt oder nicht gekoppelt sind.

6. Diagnosekit nach einem der Ansprüche 1 bis 5, **dadurch gekennzeichnet, dass** die Nitrocellulose-Membran, die auf einem Teststäbchenträger aufgebracht ist, an einem ersten Ende mit einer Membran und an einem zweiten Ende mit einem Absorptionspapier in Kontakt steht und die beiden getrennten Testzonen in Migrationsrichtung der Flüssigkeit aufeinander folgend aufweist, wobei gegebenenfalls eine Kontrollzone die beiden Testzonen trennen kann.

7. Diagnosekit nach Anspruch 6, **dadurch gekennzeichnet, dass** die Kontrollzone ausgehend von einem beliebigen, vorzugsweise mittels kolloidaler Goldpartikel, markierten Proteinpräparat und noch mehr bevorzugt ausgehend von einem Rinderserumalbumin oder RSA, das an kolloidale Goldpartikel gekoppelt ist, erhalten wird.

8. Diagnosekit für die gleichzeitige quantitative Bestimmung von β-Lactamen, Tetracyclinen und Sulfamiden nach den Ansprüchen 1 oder 2, **dadurch gekennzeichnet, dass** die Reaktionsmischung des Weiteren einen Antikörper umfasst, der für die Erkennung von Sulfamiden spezifisch ist und vorzugsweise direkt oder indirekt mittels kolloidaler Goldpartikel markiert ist, und dass das Auffangsystem des Weiteren ein Proteinpräparat umfasst, das an die Sulfamide konjugiert und auf der Nitrocellulose-Membran immobilisiert ist.

9. Diagnosekit für die gleichzeitige quantitative Bestimmung von β-Lactamen, Tetracyclinen und Sulfamiden nach Anspruch 8, **dadurch gekennzeichnet, dass** die Nitrocellulose-Membran, die auf einem Teststäbchenträger aufgebracht ist, an einem ersten Ende mit einer Membran und an einem zweiten Ende mit einem Absorptionspapier in Kontakt steht und die drei getrennten Testzonen sowie eine Kontrollzone, die aufeinander folgend in Migrationsrichtung der Flüssigkeit angeordnet sind, oder zwei Kontrollzonen, die jeweils alternativ zwischen zwei Testzonen angeordnet sind, aufweist.

10. Diagnosekit für die gleichzeitige quantitative Bestimmung von Analyten, die Antibiotika aus verschiedenen Klassen, mindestens aus den β-Lactamen, den Tetracyclinen und den Sulfamiden, entsprechen, **dadurch gekennzeichnet, dass** es Folgendes umfasst:
- einerseits eine einzige Reaktionsmischung, enthaltend mindestens einen ersten Rezeptor, der für das Erkennen von β-Lactamen spezifisch ist, einen zweiten Rezeptor, der auf spezifische und kompetitive Weise ein Tetracyclin und ein biotinyliertes Nucleinsäurefragment erkennt, wobei die beiden Rezeptoren vorzugsweise mittels kolloidaler Goldpartikel, entweder direkt oder indirekt mittels eines Antikörpers oder mittels eines an das Protein A gebundenen Antikörpers, markiert sind, sowie einen Antisulfamid-Antikörper sowie ein freies Sulfamid-Analogon, das entweder direkt oder indirekt mit kolloidalem Gold markiert ist;
- und andererseits ein Auffangsystem in Form eines festen Trägers, umfassend eine Nitrocellulose-Membran, auf der an drei bekannten und getrennten Stellen jeweils ein Antibiotikum mit β-Lactamkern, ein Avidin und ein gegen den Antisulfamid-Antikörper gerichteter Antikörper gebunden sind;
so dass die auf dem Auffangsystem mit drei Testzonen nachgewiesene Markierungsintensität auf unabhängige Weise von einer kompetitiven Erkennung von jedem Antibiotikum resultiert.

11. Diagnosekit nach einem der vorhergehenden Ansprüche, **dadurch gekennzeichnet, dass** die Probe im Wesentlichen flüssig ist und ausgehend von Milch, Honig, Fleisch, Eiern oder biologischen Flüssigkeiten erhalten wird.

12. Diagnosekit nach einem der vorhergehenden Ansprüche, **dadurch gekennzeichnet, dass** die für die quantitative Bestimmung der β-Lactame oder Tetracycline verwendeten Rezeptoren jeweils die Rezeptoren BlaR bzw. TetR sind, die ausgehend von bekannten Mikroorganismenklassen isoliert werden, vorzugsweise ausgehend von *Staphylococcus* aureus für BlaR und von Plasmid pSC101 von E. coli 600 für TetR.

13. Verfahren zur Anwendung eines Diagnosekits nach einem der vorhergehenden Ansprüche, **gekennzeichnet durch** die folgenden Schritte:
- die vorher beschriebene Reaktionsmischung wird mit einer zu charakterisierenden Probe in Kontakt gebracht, um eine Lösung zu erhalten, die man bei einer Temperatur im Bereich zwischen 30°C und 50°C 3 bis 15 Minuten lang inkubieren lässt;
- ein das vorher beschriebene Auffangsystem tragendes Teststäbchen wird in die erhaltene Lösung getaucht und man lässt 3 bis 15 Minuten lang inkubieren;
- das Ergebnis auf dem Teststäbchen wird entweder visuell mit dem Auge oder mittels eines optischen Teststäbchenlesers interpretiert.
